(19) Europäisches Patentamt / European Patent Office / Office européen des brevets

(11) **EP 2 236 206 B1**

(12) **EUROPEAN PATENT SPECIFICATION**

(45) Date of publication and mention
of the grant of the patent:
**09.05.2012 Bulletin 2012/19**

(51) Int Cl.:
***B01L 3/00*** (2006.01)

(21) Application number: **10158372.2**

(22) Date of filing: **30.03.2010**

(54) **Device for the separation of the DNA molecules having different lengths**

Vorrichtung zur Trennung von DNA-Molekülen verschiedener Länge

Dispositif de séparation de molécules d'ADN de longeurs différentes

(84) Designated Contracting States:
**AT BE BG CH CY CZ DE DK EE ES FI FR GB GR
HR HU IE IS IT LI LT LU LV MC MK MT NL NO PL
PT RO SE SI SK SM TR**

(30) Priority: **01.04.2009 IT TO20090253**

(43) Date of publication of application:
**06.10.2010 Bulletin 2010/40**

(60) Divisional application:
**11162942.4 / 2 338 598**

(73) Proprietor: **Universita' Degli Studi Di Genova
16126 Genova (IT)**

(72) Inventors:
• **Repetto, Luca
I-16134, GENOVA (IT)**
• **Firpo, Giuseppe
I-16157, GENOVA (IT)**
• **Boragno, Corrado
I-16134, GENOVA (IT)**
• **Valbusa, Ugo
I-16136, GENOVA (IT)**
• **Angeli, Elena
I-16158, GENOVA (IT)**

(74) Representative: **Fassio, Valeria et al
Jacobacci & Partners S.p.A.
Corso Emilia, 8
10152 Torino (IT)**

(56) References cited:
WO-A1-2008/134363    WO-A2-2008/071982
DE-A1-102006 035 072    US-A1- 2002 160 356

## Description

The present invention refers to a device and to a method for the separation of DNA molecules having different lengths.

Devices for the separation of DNA molecules are known based upon the known *entropic recoil* method, as described for example, in Cabodi et al., "Entropic Recoil Separation of Long DNA molecules", Anal. Chem., 2002, 74, 5169-5174 .

Such devices comprise a nano-structured region for confining DNA molecules, in particular comprising an *array* of small columns with nanometric diameter and spacing.

The nano-structures must have dimensions smaller than the dimensions of the DNA molecules in their supercoiled configuration.

The confinement region, used to induce variations in the configuration of the involved DNA molecules is obtained with a rigid material, (preferably silicon nitride), which does not allow the geometry and the dimensions of the region to vary.

The confinement thus makes the molecules abandon their supercoiled configuration promoting a "low entropy" configuration in which the chain of DNA is stretched.

In such devices, as mentioned above, electric fields are applied making it easier for the DNA molecules to enter the nano-structured confinement region. In particular, pulsed electric fields are applied having variable duration at the ends of the confinement region.

In order to make it possible to completely insert molecules with long chains, i.e. having a number of bases preferably greater than 30.000, in such a nano-structured region, the electric field must be applied for a time period longer than that necessary to insert shorter chains.

The time necessary for the insertion is related to the intensity of the electric field through the following relationship:

$$ t_c = \frac{L}{\mu_{nano}} E $$

where $t_c$ is the duration of the impulse of the applied electric field, E is the intensity of the electric field, L is the length of the DNA molecule that must be completely inserted in the confinement region, $\mu_{nano}$ is the mobility of the DNA molecules (in the confinement region) and is mainly related to the geometric dimensions of the channels.

If a chain is not completely inserted in the confinement region and/or is not able to pass through it, when the electric field is turned off, it comes out from such a nano-structured region and takes up a supercoiled configuration again due to an entropic force acting upon the molecule itself.

Such devices, however, have multiple drawbacks.

Firstly, the rigidity of the confinement region means that the separation is only related to the characteristics of the applied electric field (intensity E and time duration of the impulse $t_c$) and not to the mobility $\mu_{nano}$ of the DNA molecules inside the confinement region, which remains constant. Moreover, the use of rigid materials, such as silicon nitride, silicon oxide, fused quartz, etc. requires manufacturing processes which are costly for making a single device.

Secondly, it is necessary to apply the electric field for a long time in order to ensure a complete insertion of DNA molecules with a long chain into the confinement region.

There are also other known types of devices for the separation of DNA molecules that are based upon the use of closed channels of sub-micrometric dimensions made from elastomeric material, and are therefore mechanically deformable, like for example described in Huh et al., "Tuneable elastomeric nanochannels for nanofluidic manipulation", Nature Materials, vol. 6, June 2007, 424-428.

Such devices are manufactured with an elastomeric material the elasticity of which makes it possible to vary the section and the geometry of channels of the confinement region so as to control the passage of the DNA molecules. However, for the separation of the DNA molecules pulsed electric fields are not used. The separation occurs solely thanks to the passage of the molecules through the confinement region, and therefore the *entropic recoil* mechanism linked to the application of pulsed electric fields is not used. The only electric fields present within such devices are used solely for transporting the molecules in the confinement region, but they are not used in a pulsed way for performing the separation according to the *entropic recoil* mechanism.

The mechanical deformation of the structure is obtained by resting "small weights" on it, having different nominal mass values, which exert a mechanical force on the confinement region causing it to deform.

In such a way, it is not possible to vary the magnitude of the force applied to the structure of the device continuously, since this is a function of the discrete mass values of the various "small weights". Moreover, it is not possible to control the deformation very precisely.

Such devices are manufactured by using a cost-effective method, the randomised *nanocrack* method, which does not however make it possible to control the number of channels manufactured in the confinement region and only makes it possible to manufacture channels with an isosceles triangle-shaped section.

Document WO 2008/134363 discloses tunable elastomeric nano-channels for nano-fluidic manipulation comprising a transport component, such as electric field, to transport molecules through the nano-channels wherein the cross-sectional size of the nano-channels can be modulated in response to compressive forces applied perpendicular to the nano-channels.

The purpose of the present invention is therefore that of proposing a device for the separation of DNA molecules that does not require the application of the electric fields for long time periods, which is simple and cost-effective to manufacture and that makes it possible to control the passage of the DNA molecules through the confinement region in a precise manner.

These and other purposes are achieved with a device for the separation of DNA molecules the characteristics of which are defined in claim 1.

Particular embodiments form the subject of the dependent claims, the content of which should be considered to be an integral and integrating part of the present description.

Further characteristics and advantages of the invention shall become clearer from the following detailed description, given purely as an example and not for limiting purposes, with reference to the attached drawings, in which:

- figure 1 is a top view of a portion of the device, containing the confinement region, according to the invention;
- figure 2 is a side section view, in the direction indicated by the arrow A of figure 1, of a portion of the device according to the invention;
- figure 3 is a top view of the device according to the invention;
- figure 4 is a side section view of a deformation system of the piezoelectric type according to the invention;
- figure 5 is a side section view of a deformation system of the pneumatic type according to the invention;
- figure 6 is a side section view of a deformation system of the magnetic type according to the invention.

In brief, the device according to the invention comprises:

- a confinement region made up of channels having sub-micrometric dimensions manufactured with elastomeric materials, which can be deformed as a function of an applied piezoelectric, pneumatic or magnetic stimulation, so as to have adjustable sections;
- electrodes intended to be connected to systems for generating pulsed electric fields of a time duration which can be adapted to the length of the molecules to be separated;
- systems for the deformation of the confinement region integrated in the device for separating DNA molecules, in particular piezoelectric, pneumatic or magnetic devices.

The device according to the invention is based upon the application of the *entropic recoil* mechanism and *entropic trapping* mechanism (per se known and not disclosed in the following) to a polymeric structure with an adjustable section in which the section of the confinement region during the application of pulsed electric fields is varied.

This makes it possible to increase the flexibility of the device and its efficiency; the device indeed makes it possible to vary the section of the nano-channels in a continuous way, differently from that manufactured by Huh et al. in "Tuneable elastomeric nanochannels for nanofluidic manipulation", Nature Materials, vol. 6, June 2007, 424-428.

The device according to the invention makes it possible to continuously deform the section of the nano-channels used for the confinement, and consequently also the mobility of the molecules inside such a region.

Moreover, the continuous variation of the section makes it possible to exploit the different mobility undergone by the DNA molecules in different confinement conditions, in the best way possible.

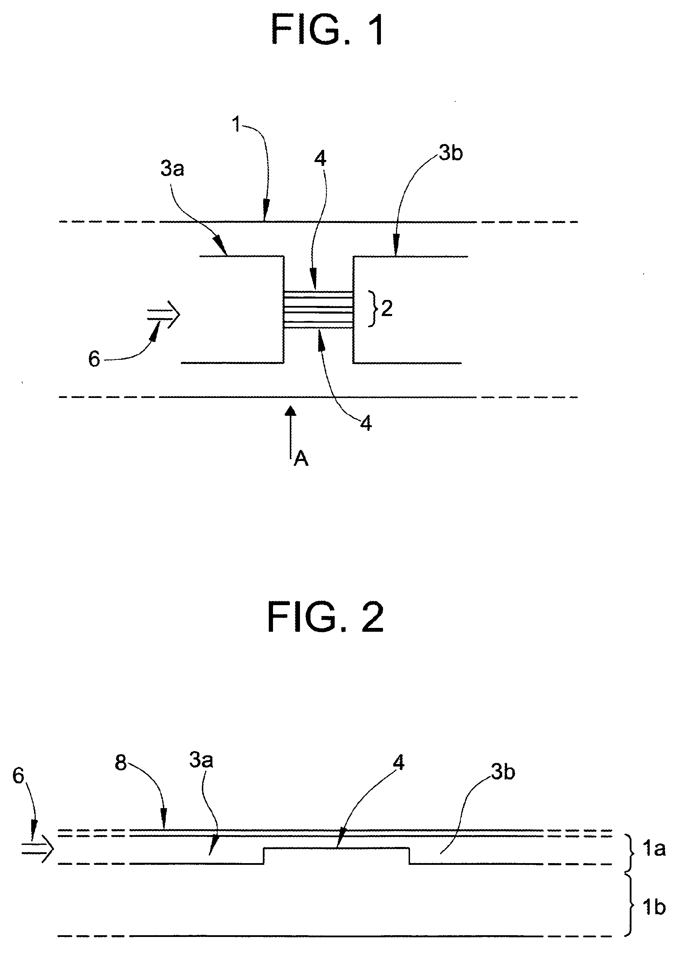

Figure 1 illustrates a top view of a portion of a device 1 for separating DNA molecules according to the invention.

Such a device 1 comprises two micro-channels 3a and 3b separated by a variable distance of between 10 and 500 micron, according to the length of the molecules to be separated, which are connected by a plurality of nano-channels 4 with sub-micrometric dimensions which represent the confinement region 2.

Such nano-channels 4 preferably have a triangular section, with a base of a width ranged between 100nm-2$\mu$m, and a height ranged between 50-400nm. Advantageously, the nano-channels 4 are spaced apart by a distance within the range of 300nm-10$\mu$m.

A flow of DNA molecules moves along the direction represented by the arrow 6; in particular the flow is transported by the first micro-channel 3a towards the nano-channels 4, and then continues coming out from the nano-channels 4 towards the second micro-channel 3b.

The dimensions of the nano-channels 4 are related to the dimensions of the DNA molecules intended to be separated. However, controlling the reciprocal distance between the nano-channels 4, having the same width, makes it possible to increase their number, and thus to process a higher number of DNA molecules increasing the quantity of molecules separated per unit time.

Such nano-channels 4 are used to confine long DNA molecules inside the nano-channels 4 themselves, so as to modify their configuration and reduce the configuration entropy.

This mechanism of reducing the entropy is associated with the application of electric fields that make it possible for the molecules to enter into the region with low entropy. The time necessary for such molecules to be inserted into the region with low entropy depends on the length of the molecules themselves.

The device 1 is made from elastomeric materials, such as, for example, polydimethylsiloxane (PDMS), through a two-stage replication process described hereby.

A silicon master is machined through conventional photolithographic techniquess so as to obtain a microfluidic structure on the surface, described hereafter, for managing the solution containing DNA molecules to be led near to the confinement region 2. Such a confinement region 2 is obtained by etching the nano-channels 4 into the master, through a focused ion beam.

The use of an ion beam is of crucial importance because it makes it possible, in a very accurate manner, to make nano-channels 4 having precise and controlled dimensions, having sections of various shapes (for example triangular, rectangular, etc.) whose ratios between the dimensions can be varied at will. Furthermore, thanks to the use of the ion beam it is possible to make nano-channels 4 at variable section (lengthwise), that is the nano-channels 4 comprising a succession of regions of different section, for exploiting entropic effects also within the neon-channels 4 themselves. This allows to amplify the effects of conformational variation of the molecules also within the nano-channels 4 and to increase the efficiency of the separation mechanism, according to the known *entropic trapping* method. Only a manufacturing made through the use of an ion beam allows such a flexibility.

At this point, a single layer of silane molecules, such as for example, tridecafluoro-1,1,2,2-tetrahydrooctyltrichlorosilane is deposited on the surface of the silicon master, for example through vapour phase deposition in order to reduce the surface energy of the mould and to make the separation of the subsequent replica easier.

The master is used, through the known method of "replica molding", to manufacture a first model made with a double layer of polymers.

On the single silane layer a first surface layer is deposited (having, for example, a variable thickness of between 10 and 50 micron) preferably of h-PDMS, material proposed by Schmid, H.; Michel, B. in "Siloxane Polymers for high-resolution, high-accuracy soft-lithography, Macromolecules (2000), 33, 3042-3049. Subsequently, a second layer is deposited (having, for example, a variable thickness of between 2 and 4mm) of PDMS.

The two layers mainly differ in their elastic properties, since the module of Young of the h-PDMS is about three times greater than that of the PDMS. The greater rigidity of the h-PDMS makes it possible to make polymer replicas having geometric and dimensional characteristics which are more faithful to the structures made on the silicon master with respect to replicas made in PDMS.

At this point, the first model is separated from the silicon master and on the layer of h-PDMS an oxidation process with oxygen plasma is carried out, necessary for depositing a single layer of silane, with the same technique used for the silicon master.

The first model is used, at this stage, for manufacturing, again through the method of "replica molding", for producing a second model, positive and similar to the silicon master.

A first layer of h-PDMS and a second layer of PDMS are thus deposited on the silane monolayer in succession.

Hereafter, the second model is separated from the first model and the layer of PDMS constitutes the base of the device whereas the nano-channels 4 are formed in the layer h-PDMS.

Advantageously, the PDMS and the h-PDMS are obtained in a *per se* known way through mixtures of composite polymers.

The nano-channels 4 make the DNA molecules deform so as to cross them.

In order to confine the molecules the nano-channels 4 must be closed.

Such a known closing process is called the "bonding" process and it exploits the exposure of the h-PDMS surface to an oxygen plasma.

Figure 2 illustrates a side section view, in the direction indicated by the arrow A in figure 1, of a portion of the device 1.

The nano-channels 4, made in the layer of h-PDMS, indicated with reference numeral 1a, are closed with a thin layer of polymeric material 8, such as, for example, PDMS or h-PDMS, chemically bonded through exposure of the layer of h-PDMS 1a, in which the nano-channels 4 are made, to oxygen plasma.

The h-PDMS layer 1a is placed on the base PDMS layer, indicated with reference numeral 1b.

As an alternative to using the layer of polymeric material 8, it is possible to use a thin glass slab, or a glass slab coated with PDMS or a glass slab coated with h-PDMS.

Both the PDMS and the h-PDMS present on the glass slab have methyl groups (-CH$_3$) on the surface, which thanks to the bombardment of the ions that occurs inside a chamber in which the oxygen plasma is formed, become hydroxyl groups (-OH). Such groups lead to the formation of covalent bonds (O-Si-O) with the h-PDMS layer 1a of the device 1, which make it possible to seal the nano-channels 4 so as to obtain closed structures. In the case in which the glass slab that is not coated with PDMS or h-PDMS is used, Si-O-Si bonds are directly formed.

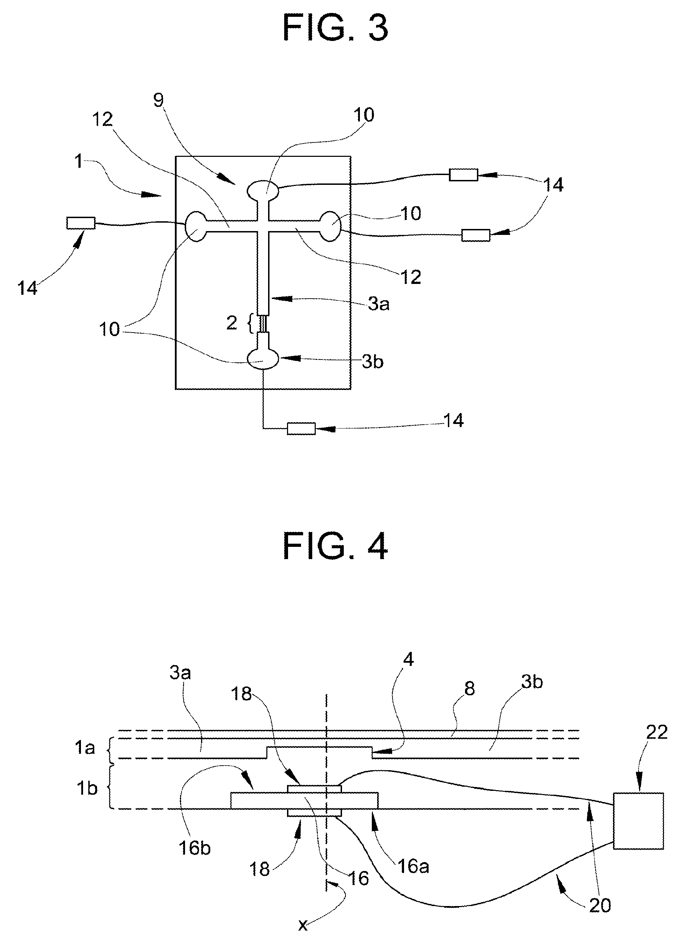

A microfluidic structure 9 that makes it possible to transport the solution necessary for the device itself to operate is illustrated, in a top schematic view, in figure 3.

The solution advantageously comprises a saline solution, such as for example, Tris-Borate and ethylenediaminetetraacetic acid, and small amounts, in the order of picomoles and nanomoles, of DNA molecules having different lengths.

The microfluidic structure 9 comprises four reservoirs 10 positioned at the ends of a Latin cross and connected, through respective micro-channels 12, to the micro-channels 3a and 3b which transport the solution containing the DNA molecules to the confinement region 2, which is in the branch of said Latin cross with greater length. The reservoirs 10 are connected to respective electrodes 14 for the application of the pulsed electric fields with a variable duration to the confinement region 2. The electrodes 14 are connected to voltage generation means such as a voltage generator (per se known and not shown in the figure) for applying pulsed electric field.

As an alternative to the Latin cross configuration, other arrangements can be used, like for example that illustrated in the aforementioned article by Huh et al.

In particular, the Latin cross configuration has the advantage of allowing the fractioning of the solution acting on the application of different electric fields to the various reservoirs; such a method is of the *per se* known type.

The device 1 is associated with a device for deforming the confinement region 2.

Such a deformation device can comprise a piezoelectric device, to carry out deformations through the use of electric fields, or a pneumatic device or a magnetic device, integrated in the device 1 as described hereafter.

Figure 4 illustrates a side section view of a deformation device of the piezoelectric type.

A disk 16 made from piezoelectric material is inserted in the PDMS layer 1b of the device 1, below the confinement region 2, and comprises for example, a block of lead zirconate titanate (PZT).

Alternatively, more complex materials are used like those proposed by Kim, K. Y.; Park, K. H.; Park, H. C.; Goo, N. S.; Yoon, K. J. in "Performance evaluation of lightweight piezo-composite actuators", Sensors and Actuators A 120, (2005), 123-129. Such materials, with the same applied voltage, allow greater deflections of the PDMS layer 1b and of h-PDMS 1a.

The disk 16 must be inserted in the device 1 so that the deformation of the PDMS layer 1b is parallel to the axis X of the disk 16 and makes it possible to deform the nano-channels 4 in a direction which is transverse with respect to their axis.

The disk 16 has a lower face 16a and an upper face 16b to which two respective electrodes 18 are coupled, connected to the respective conducting wires 20 which make it possible to apply an alternating voltage (for example, having a square-shaped wave of adjustable frequency) with a variable intensity, through a voltage source 22.

The dependency of the deflection of the piezoelectric material of the disk 16 upon the intensity of the applied voltage was shown, for example, by Kim, J.-H.; Kang, C. J.; Kim, Y.-S. in "A disposable polydimethylsiloxane-based diffuser micropump actuated by piezoelectric-disc", Microelectronic Engineering, 71, (2004), 119-124.

A careful modulation of the voltage applied by the source 22 allows an accurate control of the deflection of the disk 16, and consequently of the deformation of the section of the nano-channels 4, in a much more accurate and continuous way with respect to that proposed in the aforementioned article by Huh et al.

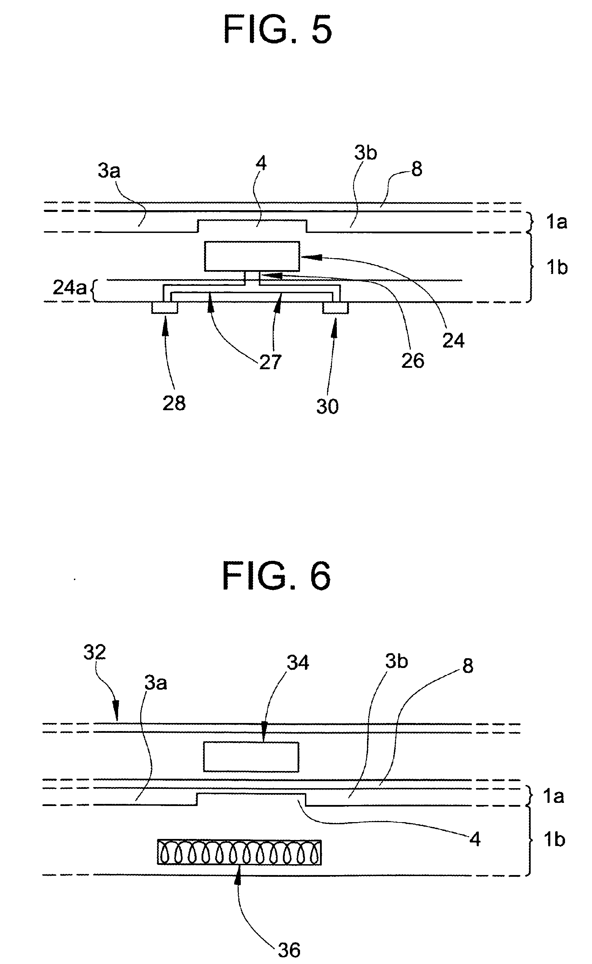

Figure 5 illustrates a side section view of a deformation device of the pneumatic type.

Inside the PDMS layer 1b of the device 1 a pneumatic "expansion chamber" 24 is created below the confinement region 2. Such an "expansion chamber" 24, made from PDMS, is connected through a hole 26 to a layer 24a made from a rigid polymer such as, for example, PMMA (polymethylmethacrylate), which does not deform when subjected to pressure. The PDMS, a more elastic material, is deformed when subjected to pressure by a gas inserted into the "expansion chamber" 24.

Connection channels 27 are also made to bring a pressurized gas, such as for example air, to the "expansion chamber" 24. Such a gas enters the connection channels 26 through an inlet electrovalve 28 and comes out through an outlet electrovalve 30.

The deformation of the "expansion chamber" 24 makes it possible to deform the layers of PDMS 1b and h-PDMS 1a, and thus also the nano-channels 4, which are above the "expansion chamber" 24.

Figure 6 illustrates a side section view of a deformation device of the magnetic type.

A layer of supporting material 32, for example of PDMS, is deposited or bonded through an exposure to an oxygen plasma (as described above) on the layer of polymeric material 8. Inside this supporting layer 32 a magnet 34 is arranged above the confinement region 2. An electromagnet 36 is arranged in the PDMS layer 1b under the confinement region 2. The electromagnet 36 is supplied with a current so as to create an attractive force with respect to the magnet 34. In such a way, the layers of PDMS 1b, h-PDMS 1a and the nano-channels 4 interposed between the magnet 34 and the electromagnet 36 are deformed in a controlled manner.

The magnet 34 comprises, for example, a steel disk, or a block of composite material made up of particles of magnetic material, like for example NdFeB, dispersed in a polymer matrix like for example PDMS. Examples of such materials are shown in the following article: Yamahata, C; Lotto, C., Al-Assaf, E.; Gijs, M. A. M. in "A PMMA valveless micropump using electromagnetic actuation", Microfluid Nanofluid, (2005), 1, 197-207.

Alternatively, instead of the magnet 34 and of the electromagnet 36, an electrovalve of the *per se* known type having a mobile portion can be located in the PDMS layer 1b under the confinement region 2. By activating the electrovalve, the mobile part deforms the layers of PDMS 1b, of h-PDMS 1a and the nano-channels 4 positioned above it.

The device according to the invention is advantageous since it makes it possible to simultaneously exploit different separation mechanisms, one related to the dimensions of the confinement area and one related to the application of electric fields with variable intensity and duration, in a single device.

Moreover, a low-cost method - the model-making process, suitable for mass producing devices - is used to make the device. Moreover, the cost related to the use of an onerous technology like the use of a focused ion beam for manufacturing the nano-channels 4 on the silicon master, is split among a very high number of models.

The use of a thin glass slab, or of polymeric material, and of an oxygen plasma exposure for closing the nano-channels 4 is also a cost-effective process, which does not require complicated apparatuses and is desirable for an efficient industrial process in series.

Of course, without affecting the principle of the invention, the embodiments and the manufacturing details can be widely varied with respect to what has been described and illustrated purely as a non limiting example, without thereby departing from the scope of protection of the present invention defined in the attached claims.

## Claims

1. Device (1) for the separation of the DNA molecules comprising:

   - an elastomeric confinement region (2) adapted to receive said DNA molecules;
   - a plurality of electrodes (14) and voltage generation means connected thereto, said voltage generation means being arranged to apply to said confinement region (2) electric fields, so as to make easier the entry of DNA molecules in the confinement region (2);

   **characterized by** the voltage generation means being arranged to apply pulsed electric fields and in that the device further comprises a piezoelectric, or pneumatic or magnetic deformation device (16; 24; 34, 36) of the confinement region (2).

2. Device according to claim 1, wherein said confinement region (2) comprises a plurality of nano-channels (4) arranged to be crossed by said DNA molecules.

3. Device according to claim 2, comprising a first micro-channel (3a) and a second micro-channel (3b) connected each other by said plurality of nano-channels (4), said first micro-channel (3a) being arranged to transport the DNA molecules towards the nano-channels (4) and said second micro-channel (3b) being arranged to collect the DNA molecules that come out from the nano-channels (4).

4. Device according to any of the preceding claims, further comprising a microfluidic structure (9) arranged to lead the DNA molecules to the confinement region (2).

5. Device according to any of the preceding claims, wherein the deformation device (16, 24; 34, 36) comprises a disk of piezoelectric material (16) inserted below the confinement region (2), two electrodes (18) coupled to the disk (16) for the application of an alternating voltage so as to produce a deformation of the disk (16), said deformation of the disk (16) causing a deformation of the confinement region (2).

6. Device according to any of the preceding claims, wherein the deformation device (16, 24; 34, 36) comprises a pneumatic expansion chamber (24) placed below the confinement region (2) and arranged to receive a pressurized gas so that the expansion chamber (24) deforms, said deformation of the expansion chamber (24) causing a deformation of the confinement region (2).

7. Device according to any of the preceding claims, wherein the deformation device (16; 24; 34, 36) comprises a magnet (34) placed above the confinement region (2), and an electromagnet (36) placed below the confinement region (2), said electromagnet (36) being arranged to exercise an attractive force towards the magnet (34) so as to cause a deformation of the confinement region (2).

## Patentansprüche

1. Vorrichtung (1) zum Trennen von DNA-Molekülen, wobei die Vorrichtung umfasst:

   - einen elastomeren Einengungsbereich (2), der so ausgebildet ist, dass er die DNA-Moleküle empfängt;
   - eine Vielzahl von Elektroden (14) mit einer Spannungserzeugungs-Einrichtung, die mit diesen verbunden ist, wobei sie so angeordnet ist, dass sie elektrische Felder an den Einengungsbereich (2) legen, um den Eintritt von DNA-Molekülen in den Einengungsbereich (2) zu erleichtern;

   **dadurch gekennzeichnet, dass** die Spannungserzeugungs-Einrichtung so angeordnet ist, dass sie gepulste elektrische Felder anlegt, und dass die Vorrichtung weiters eine piezoelektrische oder pneumatische oder magnetische Verformungseinrichtung (16; 24; 34, 36) für den Einengungsbereich (2) umfasst.

2. Vorrichtung gemäß Anspruch 1, wobei der Einengungsbereich (2) eine Vielzahl von Nano-Kanälen (4) umfasst, die so angeordnet sind, dass sie von den DNA-Molekülen durchlaufen werden.

3. Vorrichtung gemäß Anspruch 2, wobei die Vorrichtung einen ersten Mikro-Kanal (3a) sowie einen zweiten Mikrokanal (3b) umfasst, die miteinander über die Vielzahl von Nano-Kanälen (4) verbunden sind, wobei der erste Mikrokanal (3a) so angeordnet ist, dass er die DNA-Moleküle zu den Nano-Kanälen (4) transportiert, und der zweite Mikro-Kanal (3b) so angeordnet ist, dass er die DNA-Moleküle sammelt, die von den Nano-Kanälen (4) kommen.

4. Vorrichtung gemäß irgendeinem der bisherigen Ansprüche, wobei die Vorrichtung weiters eine Mikrofluid-Struktur (9) umfasst, die so angeordnet ist, dass sie die DNA-Moleküle zum Einengungsbereich (2) leitet.

5. Vorrichtung gemäß irgendeinem der bisherigen Ansprüche, wobei die Verformungseinrichtung (16, 24; 34, 36) eine Scheibe aus einem piezoelektrischen Material (16), die unterhalb des Einengungsbereiches (2) eingesetzt wird, sowie zwei Elektroden umfasst (18), die mit der Scheibe (16) verbunden sind, um eine Wechselspannung anzulegen, um eine Verformung der Scheibe (16) zu erzeugen, wobei die Verformung der Scheibe (16) eine Verformung des Einengungsbereichs (2) hervorruft.

6. Vorrichtung gemäß irgendeinem der bisherigen Ansprüche, wobei die Verformungseinrichtung (16, 24; 34, 36) eine pneumatische Expansionskammer (24) umfasst, die unterhalb des Einengungsbereichs (2) angeordnet und so ausgebildet ist, dass sie ein unter Druck stehendes Gas so aufnimmt, dass sich die Expansionskammer (24) verformt, wobei die Verformung der Expansionskammer (24) eine Verformung des Einengungsbereichs (2) hervorruft.

7. Vorrichtung gemäß irgendeinem der bisherigen Ansprüche, wobei die Verformungseinrichtung (16; 24; 34, 36) einen Magnet (34), der oberhalb des Einengungsbereichs (2) angeordnet ist, sowie einen Elektromagnet (36) umfasst, der unterhalb des Einengungsbereichs (2) angeordnet ist, wobei der Elektromagnet (36) so angeordnet ist, dass er gegenüber dem Magnet (34) eine Anziehungskraft ausübt, um eine Verformung des Einengungsbereichs (2) hervorzurufen.

**Revendications**

1. Dispositif (1) de séparation de molécules d'ADN comprenant :

    - une région de confinement en élastomère (2) adaptée pour recevoir lesdites molécules d'ADN ;
    - une pluralité d'électrodes (14) et moyens de génération de tension électrique connectés à celles-ci, lesdits moyens de génération de tension étant adaptés pour appliquer à ladite région de confinement (2) des champs électriques, afin de faciliter l'entrée de molécules d'ADN dans la région de confinement (2) ;

**caractérisé en ce que** le moyen de génération de tension est adapté pour appliquer des champs électriques pulsés et **en ce que** le dispositif comprend en outre un dispositif de déformation piézoélectrique ou pneumatique ou magnétique (16 ; 24 ; 34 ; 36) de la région de confinement (2).

2. Dispositif selon la revendication 1, dans lequel ladite région de confinement (2) comprend une pluralité de nanocanaux (4) adaptés pour être traversés par lesdites molécules d'ADN.

3. Dispositif selon la revendication 2, comprenant un premier microcanal (3a) et un deuxième microcanal (3b) reliés entre eux par ladite pluralité de nanocanaux (4), ledit premier microcanal (3a) étant adapté pour transporter les molécules d'ADN vers le nanocanaux (4) et ledit deuxième microcanal (3b) étant adapté pour collecter les molécules d'ADN qui sortent des nanocanaux (4).

4. Dispositif selon l'une quelconque des revendications précédentes, comprenant en outre une structure microfluidique (9) adaptée pour amener les molécules d'ADN jusqu'à la région de confinement (2).

5. Dispositif selon l'une quelconque des revendications précédentes, dans lequel le dispositif de déformation (16, 24 ; 34, 36) comprend un disque en matériau piézoélectrique (16) inséré sous la région de confinement (2), deux électrodes (18) couplées au disque (16) pour l'application d'une tension alternative destinée à produire une déformation du disque (16), ladite déformation du disque (16) provoquant une déformation de la région de confinement (2).

6. Dispositif selon l'une quelconque des revendications précédentes, dans lequel le dispositif de déformation (16, 24 ; 34, 36) comprend une chambre d'expansion pneumatique (24) placée sous la région de confinement (2) et adaptée pour recevoir un gaz sous pression destiné à déformer la chambre d'expansion (24), ladite déformation de la chambre d'expansion (24) provoquant une déformation de la région de confinement (2).

7. Dispositif selon l'une quelconque des revendications précédentes, dans lequel le dispositif de déformation (16 ; 24 ; 34, 36) comprend un aimant (34) placé au-dessus de la région de confinement (2), et un électroaimant (36) placé sous la région de confinement (2), ledit électroaimant (36) étant adapté pour exercer une force d'attraction vers l'aimant (34) afin de provoquer une déformation de la région de confinement (2).

# FIG. 1

# FIG. 2

# FIG. 3

# FIG. 4

# FIG. 5

# FIG. 6

## REFERENCES CITED IN THE DESCRIPTION

**Patent documents cited in the description**

- WO 2008134363 A

**Non-patent literature cited in the description**

- **Cabodi et al.** Entropic Recoil Separation of Long DNA molecules. *Anal. Chem.,* 2002, vol. 74, 5169-5174
- **Huh et al.** Tuneable elastomeric nanochannels for nanofluidic manipulation. *Nature Materials,* June 2007, (6), 424-428
- **Schmid, H. ; Michel, B.** Siloxane Polymers for high-resolution, high-accuracy soft-lithography. *Macromolecules,* 2000, vol. 33, 3042-3049
- **Kim, K. Y. ; Park, K. H. ; Park, H. C. ; Goo, N. S. ; Yoon, K. J.** Performance evaluation of lightweight piezo-composite actuators. *Sensors and Actuators A,* 2005, vol. 120, 123-129
- **Kim, J.-H. ; Kang, C. J. ; Kim, Y.-S.** A disposable polydimethylsiloxane-based diffuser micropump actuated by piezoelectric-disc. *Microelectronic Engineering,* 2004, vol. 71, 119-124
- **Yamahata, C ; Lotto, C. ; Al-Assaf, E. ; Gijs, M. A. M.** A PMMA valveless micropump using electromagnetic actuation. *Microfluid Nanofluid,* 2005, vol. 1, 197-207